# EUROPEAN PATENT SPECIFICATION

(11) **EP 1 587 522 B1**
(45) Date of publication and mention of the grant of the patent: **01.10.2008**
(21) Application number: 04700741.4
(22) Date of filing: 08.01.2004
(51) Int. Cl.: A61K 31/198

(54) **ATTAINING SEXUAL WELLNESS AND HEALTH OF THE SEXUAL VASCULAR SYSTEM WITH PROANTHOCYANIDINS**
HERSTELLUNG VON SEXUALEN WELLNESS UND GESUNDHEIT DES SEXUALASCULARSYSTEMS MIT PROCANTHOCYANDINEN.
PROANTHOCYANIDINES PERMETTANT UN BIEN-ETRE SEXUEL ET LA SANTE DU SYSTEME VASCULAIRE SEXUEL

(30) Priority: 13.01.2003 US 439737 P; 15.10.2003 US 685677
(43) Date of publication of application: 26.10.2005
(73) Proprietor: Horphag Research (Luxembourg) Holding SA, 1212 Luxembourg (LU)
(72) Inventor: ROHDEWALD, Peter, 481 Munster (DE); FERRARI, Victor, CH-3784 Feutersoey (CH)
(74) Representative: Crump, Julian Richard John
(86) International application number: PCT/IB2004/000019
(87) International publication number: WO 2004/062680

(56) References cited:
- WO-A-01/89543
- US-A1- 2002 037 862

## Description

### Field of the Invention

The invention relates to improving sexual fitness or wellness of both sexes and the health of the sexual vascular system with ingredients that include a source of proanthocyanidins, a source of arginine and, a testosterone supplement. The source of proanthocyanidins may be a botanical extract and the source of arginine may be from arginine aspartate. The testosterone supplement may be testosterone itself, a testosterone precursor, a testosterone production stimulator or a testosterone bioavailability enhancer. All or some of the ingredients may be part of a composition and/or be separate from each other within a kit.

### Discussion of Related Art

When a male is in hls early twenties, it's easy to take peak sexual performance for granted. Yet as time passes, the male body's biological system changes, and he may notice that his sexual stamina, performance and even pleasure begin to decrease. Getting "in the mood" may start to take a little effort.

Many women have problems with sex when they reach menopause and their ovaries produce smaller amounts of sex hormones. Lower levels of estrogen can make the vaginal tissue dry, and less androgen leads to less sexual desire and arousal,

One important difference affecting sexual desire is that men have levels of testosterone that are 20 to 30 times what women have. Men's testosterone levels gradually decline over time but they do not experience a drop-off as women do at menopause. In men and women, testosterone and other androgens work to increase desire.

Androgen gels and patches for women are being considered for women with sexual dysfunction. Another possibility to overcome the female androgen deficiency syndrome is to supply women with 50 mg dehydroepiandrosterone per day, which facilitates the enhanced production of testosterone, dehydrotestosterone, androstenedione and androstenediol. That improved female androgenic profile causes intense sexual thoughts and a general enhancement in mental and physical sexual arousal (Spark, R.F., 2002; Hackbert, L. and Heiman J.R., 2002).

To increase blood flow in the female genitale tissue is also useful to improve sexual wellness. The New York Times on March 25, 2003 published an article entitled "Effort to Make Sex Drug for Women Challenges Experts". According to the article, researchers found that women's sex organs are not as readily affected as men's by sildenafil, which is the active ingredient of a drug sold under the trademark VIAGRA. Blocking the same enzyme in women that normally inhibits blood flow does not increase circulation to genital tissue so drastically as in men for causing engorgement of erectile tissue.

Studies suggest that sildenafil alone does not fix female arousal problems. However, when taken together with supplemental hormones, at least one study showed that 57 percent of 202 postmenopausal women involved in a study reported improved genital sensations, compared with 43 percent of a placebo group. Forty-one percent of the sildenafil group members reported greater satisfaction with sex, compared with 27 percent in the placebo group. Although the differences between the two groups were modest, the study suggests that sildenafil could help women with healthy hormone levels and in happy relationships.

One may surmise that female sexual function is accomplished physiologically in a similar manner like in man in a way that cGMP triggers lubrication and engorgement of the clitoral tissue. The studies mentioned in the previously mentioned article suggest the possibility that when women have a healthy hormone level, such dietary supplements may help improve sexual function in women to some extent.

Another way to increase the blood flow into the female or male sexual organs is to increase the production of nitric oxide, which in turn triggers the release of cGMP. Whereas sildenafil and related substances lead to a sustained increase of blood content of the male or female sexual organs by blocking the enzymatic destruction of the vasodilating cGMP, nitric oxide produces the same increased blood volume by enhancing the production of cGMP.

As a physiological source for nitric oxide production, the aminoacid L-arginine is used. The enzyme endothelial nitric oxide synthase produces nitric oxide from L- arginine. To provide an enhanced and sustained blood flow to the sexual organs, it is first of all necessary to supplement the organism with the substrate L-arginine in sufficient quantities. However, the presence of high concentrations of L-arginine alone does not lead to a substantial higher blood flow to the sexual organs. It is necessary to stimulate additionally the endothelial nitric oxide synthase, so that nitric oxide production from L-arginine is catalyzed by the active enzyme. A potent stimulator of endothelial nitric oxide synthase is a proanthocyanidins-containing extract,

Proanthocyanidins represent a group of plant polyphenols found in roots, barks and fruits with an astringent taste. Proanthocyanidins Include the subgroups of procyanidins and prodelphinidins. Proanthocyanidins are biopolymers composed of flavan subunits. Procyanidins are composed of catechin and epicatechin units, also called monomeric procyanidins.

Proanthocyanidins are extracted from plant material by conventional methods using solvents like water, ethanol or acetone or fluid carbon dioxide. The extracts are purified by solvent/solvent extraction, ultra filtration or chromatographic procedures. The purified extracts are concentrated by solvent evaporation, freeze drying or spray drying.

A proanthocyanidin-rich extract from the bark of French maritime pine is distributed under the tradename Pycnogenol^{®} by Horphag Research, Switzerland. The extract contains 70-75% by weight proanthocyanidins and other flavanols such as catechin, epicatechin and taxifolin. Other proanthocyanidins rich extracts can be obtained from grape seeds, cones from cypress trees, cocoa beans or other plant materials. Pycnogenol^{®} pine bark extract has been shown to stimulate endothelial nitric oxide synthase and to induce vasodilation (Fitzpatrick, D.F., Bing, B., Rohdewald, P., 1998)

PCT application WO 01/89543 relates to the use of proanthocyanidins and L-Arginine or its salts for the treatment of erectile dysfunction.

### SUMMARY OF THE INVENTION

One aspect of the invention resides in the use of a product for the manufacture of a safe, natural medicament for increasing sperm quality and/or increasing fertility. It includes a preparation that is a blend of ingredients, namely, proanthocyanidins and a substrate that is a source of arginine, preferably a salt or dipeptide of L-arginine and aspartic acid, such as arginine aspartate and a testosterone supplement. When the blend Is administered, the endothelial NO-synthase is stimulated by the proanthocyanidins. Nitric oxide is released from the substrate In response to the stimulated endothelial NO-synthase enzyme, which acts as a catalyst for synthesis of the nitric oxide from the substrate. The source of arginine and proanthocyanidins are in therapeutically effective amounts to cause a sufficient amount of the nitric oxide to be released from the synthesis so that when fresh supplies are taken on a dally basis over a period of time, sexual fitness or sexual wellness improves by the end of the period of time. The blend may contain testosterone or dehydroepiandrosterone to overcome the lack of androgenic hormones thereby increasing or restoring sexual arousal and desire.

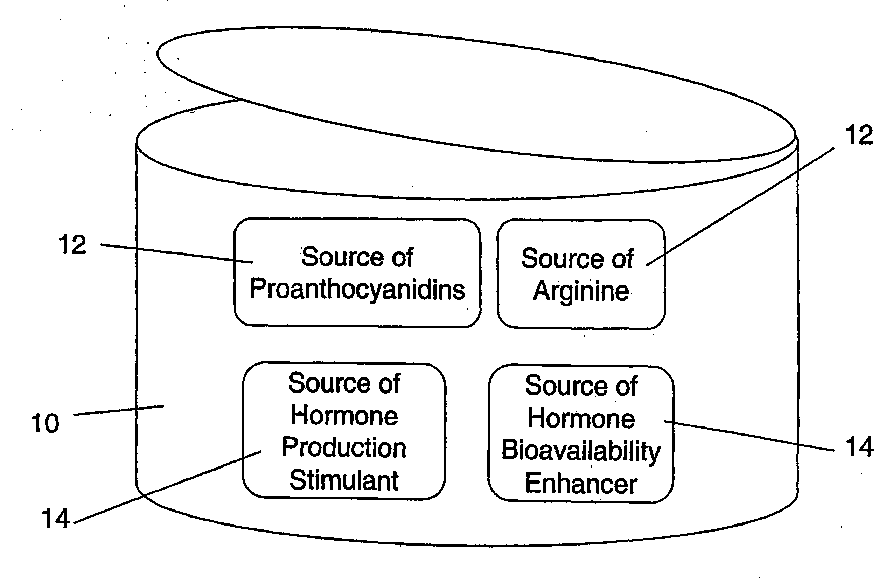

### BRIEF DESCRIPTION OF THE DRAWING

The drawing shows a kit In accordance with the Invention.

### DETAILED DESCRIPTION OF THE INVENTION

The invention pertains to the prolonged use of a blend of ingredients, namely, a source of arginine (such as L-arginine), a source of proanthocyanidins and a testosterone supplement. Preferably, the source is a salt or peptide of arginine and aspartic acid, namely, arginine aspartate. Preferably, the source of proanthocyanidins is derived from Pycnogenol^{®} or from other proanthocyanidin-containing extracts. Examples of precursors of Testosterone include dehydroepiandrosterone (DHEA) and dehydroepiandrosterone sulfate (DHEAS).

The preparation in accordance with the present invention may be in the form of a blend of ingredients, whether as a composition, taken either in tablet form or in liquid form, or as separate ingredients being in separate, distinct tablet or liquid form but packaged together in a kit 10. In the latter case, the separate ingredients 12 are taken either simultaneously, such as by mixing them together if in liquid form, or one after another if in tablet form. Additional ingredients 14 such as a hormone (testosterone )or a hormone production stimulant, a source of a hormone (testosterone) bioavailability enhancer, a precursor of a hormone may be included in the Kit 10.

### Example1:

In the department of Dr. Stanislavov and Dr. Nikolova 50 men aged between 45-60 with testosterone levels below normal were treated daily with
Pycnogenol^{®} 120 mg
L-arginine (Sargenor) 1,71g
Testosterone undecanoate 120 mg
over a period of 12 months because of secondary infertility. The patients filled a questionnaire (Global efficacy questionnaire) whether the treatment improved the erection.

Before treatment, only 10 % of the patients reported a normal erection.

**TABLE 3: Assessment of efficacy and satisfaction of treatment**

| Assessment of erection | Before treatment | After 1 month treatment with L-arginine (only) | After 3 months treatment added Pycnogenol^{®} + Andriol¹ | After 6 months treatment added Pycnogenol^{®} + Andriol¹ | After 12 months treatment added Pycnogenol^{®} + Andriol¹ |
|---|---|---|---|---|---|
| Disturbed: weakened delayed hesitating loosing | 45 (90%) | 42 (84%) NS | 30 (60%)* | 21 (42%)* | 12 (24%)** |
| Normal | 5 (10%) | 8 (16%) | 20 (40%)* | 29 (58%)* | 38 (76%)** |

| | | | | | |
|---|---|---|---|---|---|
| ¹Andriol: Testosterone undecanoate 40 mg (Organon GmbH); NS - non significant; *p<0.05; **p<o.o1 | | | | | |

After 1 month treatment with L-arginine, the number of men with a normal erection increased non-significantly to 16%. After addition of Pycnogenol^{®} and testosterone undecanoate to treatment, the percentage of men experiencing a normal erectile function increased from 40% up t 76% over the treatment period. In addition, the quality of sperm was significantly improved and 44% of the couples achieved pregnancy.

Example 2 demonstrates that men with abnormally low levels of testosterone and subsequent secondary infertility and disturbed sexual function greatly improved in terms of erectile function as well as in quality of sperms. The continuous supplementation with L-arginine together with the stimulator of endothelial nitric oxide synthase and the sex hormone testosterone was successful in improving sexual wellness and fertility.

The supplementation with L-arginine and a proanthocyanidin-containing extract on a regular basis allows healthy couples to spontaneously react to their partners stimulation. The combined supplementation with an added hormone, hormone precursor or hormone bioavailability enhancer is very useful for both sexes in case of reduced production of androgenic hormones.

After attaining the optimum level of sexual wellness and health of the sexual vascular system in accordance with the invention, missing one daily administration of the preparation of the invention as ingredients of a composition or separate ingredients within the kit will not diminish the level of sexual wellness and health of the sexual vascular back to the same level as it was prior to the taking of the present invention the first time. There will be some lingering, residual effect in the body that will carry over to the following day so that a daily administration of a single dose or serving thereafter will eventually restore the sexual wellness and health of the sexual vascular system to the same optimum level as before.

The preparation in accordance with the invention, whether the ingredients be blended together in a composition or kept separate and distinct from each yet sold together as part of a kit, may be promoted through commercially advertising the same. In this connection, the advertising may promote that the preparation enhances sexual wellness or sexual fitness and may recommend that the preparation be administered daily.

## Claims

1. Use of a preparation for the manufacture of a medicament for increasing sperm quality and/or increasing fertility by stimulating nitric oxide synthase enzyme and releasing nitric oxide, **characterized by**: administering the preparation daily over a period of time, the preparation comprising ingredients that include a substrate, a stimulator, and a testosterone supplement, the substrate being a source of arginine and, subsequently, of nitric oxide; the stimulator including proanthocyanidins to stimulate an endothelial NO-synthase enzyme, which serves as a catalyst for synthesis of the nitric oxide; the ingredients being of sufficient quantity to trigger release of an amount of nitric oxide from the synthesis sufficient to increase sperm quality and/or increase fertility by an end of the period of time.

2. The use of claim 1, wherein the medicament is for use for increasing sperm quality.

3. The use of claims 1 or claim 2, wherein the medicament is for use for increasing fertility.

4. The use of any of claims 1 to 3, wherein the testosterone supplement is provided in the form of a testosterone precursor.

5. The use of claim 4, wherein the testosterone precursor is selected from DHEA and DHEAS.

6. The use of any of claims 1 to 3, wherein the testosterone supplement is selected from a group consisting of a hormone concentration increaser that increases concentration of hormones in blood, a testosterone production stimulator that stimulates production of testosterone.

7. The use of any of claims 1 to 3, **characterized in that** the preparation also includes at least one testosterone supplement that increases a proportion of testosterone in its free form to be more bioavailable.

8. The use of any of claims 1 to 3, wherein the testosterone supplement having an ingredient selected from a group consisting of the testosterone and a stimulator of a production agent of testosterone configured to increase bioavailability of testosterone upon administration.

9. The use of any of claims 1 to 3, **characterized in that** the ingredients are blended together to constitute a composition.

10. The use of any of claims 1 to 3, **characterized in that** the ingredients are separate and distinct tablet or liquid form but packaged together as a kit.

11. The use of any previous claim, wherein said medicament also enhances sexual wellness.

## Patentansprüche

1. Verwendung eines Präparats zur Herstellung eines Medikaments zur Steigerung der Spermien-Qualität und/oder Steigerung der Fertilität durch Stimulieren von Stickoxid-Synthase-Enzym und Freisetzen von Stickoxid, **gekennzeichnet durch**: tägliches Verabreichen des Präparats über einen Zeitraum, wobei das Präparat Ingredienzien umfasst, die ein Substrat, einen Stimulator und ein Testosteron-Ergänzungsmittel inkludieren, wobei das Substrat eine Quelle für Arginin und folglich für Stickoxid ist; wobei der Stimulator Proanthocyanidine aufweist, um ein endotheliales NO-Synthase-Enzym zu stimulieren, welches als Katalysator für die Synthese des Stickoxids dient; und wobei die Ingredienzien in einer ausreichenden Menge vorliegen, um die Freisetzung einer Menge an Stickoxid aus der Synthese auszulösen, die ausreicht, um bis zum Ende des Zeitraums die Spermien-Qualität zu steigern und/oder die Fertilität zu steigern.

2. Verwendung nach Anspruch 1, wobei das Medikament zur Verwendung zur Steigerung der Spermien-Qualität dient.

3. Verwendung nach Anspruch 1 oder Anspruch 2, wobei das Medikament zur Verwendung zur Steigerung der Fertilität dient.

4. Verwendung nach einem der Ansprüche 1 bis 3, wobei das Testosteron-Ergänzungsmittel in Form eines Testosteron-Vorläufers vorgesehen ist.

5. Verwendung nach Anspruch 4, wobei der Testosteron-Vorläufer ausgewählt ist aus DHEA und DHEAS.

6. Verwendung nach einem der Ansprüche 1 bis 3, wobei das Testosteron-Ergänzungsmittel ausgewählt ist aus einer Gruppe bestehend aus einem Hormon-Konzentrations-Erhöher, der die Konzentration von Hormonen im Blut erhöht, einem Testosteron-Pruduktions-Stimulator, der die Produktion von Testosteron stimuliert.

7. Verwendung nach einem der Ansprüche 1 bis 3, **dadurch gekennzeichnet, dass** das Präparat auch mindestens ein Testosteron-Ergänzungsmittel inkludiert, das einen Anteil des Testosterons in freier Form erhöht zwecks größerer Bioverfügbarkeit.

8. Verwendung nach einem der Ansprüche 1 bis 3, wobei das Testosteron-Ergänzungsmittel ein Ingrediens ausgewählt aus einer Gruppe bestehend aus Testosteron und einem Stimulator eines Produktionswirkstoffs für Testosteron hat, der ausgelegt ist, die Bioverfügbarkeit von Testosteron nach Verabreichung zu erhöhen.

9. Verwendung nach einem der Ansprüche 1 bis 3, **dadurch gekennzeichnet, dass** die Ingredienzien zusammengemischt werden, um eine Zusammensetzung zu bilden.

10. Verwendung nach einem der Ansprüche 1 bis 3, **dadurch gekennzeichnet, dass** die Ingredienzien separate und einzelne Tabletten sind oder flüssig sind, jedoch als Set zusammen verpackt sind.

11. Verwendung nach einem vorhergehenden Anspruch, wobei das Medikament auch das sexuelle Wohlbefinden verbessert.

## Revendications

1. Utilisation d'une préparation pour la fabrication d'un médicament pour augmenter la qualité du sperme et/ou augmenter la fertilité par stimulation de l'enzyme oxyde nitrique synthétase et libération d'oxyde nitrique, **caractérisée par** : l'administration quotidienne de la préparation pendant une certaine période, la préparation comprenant des ingrédients qui comprennent un substrat, un stimulateur et un complément testostérone, le substrat étant une source d'arginine et ensuite, d'oxyde nitrique ; le stimulateur comprenant des proanthocyanidines pour stimuler l'enzyme NO-synthétase endothéliale, qui sert de catalyseur à la synthèse de l'oxyde nitrique ; les ingrédients étant en quantité suffisante pour déclencher la libération d'une quantité d'oxyde nitrique provenant de la synthèse, suffisante pour augmenter la qualité du sperme et/ou augmenter la fertilité à la fin de la période.

2. Utilisation selon la revendication 1, dans laquelle le médicament est utilisé pour augmenter la qualité du sperme.

3. Utilisation selon la revendication 1 ou 2, dans laquelle le médicament est utilisé pour augmenter la fertilité.

4. Utilisation selon l'une quelconque des revendications 1 à 3, dans laquelle le complément testostérone est procuré sous la forme d'un précurseur de testostérone.

5. Utilisation selon la revendication 4, dans laquelle le précurseur de testostérone est choisi parmi la DHEA et le DHEAS.

6. Utilisation selon l'une quelconque des revendications 1 à 3, dans laquelle le complément testostérone est choisi parmi un groupe consistant en un agent augmentant la concentration hormonale, qui augmente la concentration des hormones dans le sang, un stimulateur de la production de testostérone, qui stimule le production de la testostérone.

7. Utilisation selon l'une quelconque des revendications 1 à 3, **caractérisée en ce que** la préparation contient également, au moins un complément testostérone, qui augmente la biodisponibilité d'une partie de la testostérone sous sa forme libre.

8. Utilisation selon l'une quelconque des revendications 1 à 3, dans laquelle le complément testostérone ayant un ingrédient choisi parmi le groupe consistant en la testostérone et un stimulateur d'un agent de production de la testostérone, configuré pour augmenter la biodisponibilité de la testostérone lors de l'administration.

9. Utilisation selon l'une quelconque des revendications 1 à 3, **caractérisée en ce que** les ingrédients sont mélangés ensemble pour engendrer une composition.

10. Utilisation selon l'une quelconque des revendications 1 à 3, **caractérisée en ce que** les ingrédients sont des formes de comprimé ou liquides séparées et distinctes, mais emballées ensemble sous forme d'un kit.

11. Utilisation selon l'une quelconque des revendications précédentes, dans laquelle ledit médicament augmente le bien-être sexuel.
